(19) Europäisches Patentamt
European Patent Office
Office européen des brevets

(11) **EP 1 754 503 B1**

(12) **EUROPEAN PATENT SPECIFICATION**

(45) Date of publication and mention
of the grant of the patent:
**16.11.2011   Bulletin 2011/46**

(51) Int Cl.:
*A61M 29/00* *(2006.01)*

(21) Application number: **06254264.2**

(22) Date of filing: **15.08.2006**

(54) **Vaginal dilator**

Vaginaler dilatator

Dilatateur vaginal

(84) Designated Contracting States:
**DE**

(30) Priority:   **16.08.2005   GB 0516820**

(43) Date of publication of application:
**21.02.2007   Bulletin 2007/08**

(73) Proprietor: **Greenwood, Nicola Regan**
**London SW7 3AF (GB)**

(72) Inventor: **Greenwood, Nicola Regan**
**London SW7 3AF (GB)**

(74) Representative: **Campbell, Neil Boyd**
**Dehns**
**St Bride's House**
**10 Salisbury Square**
**London**
**EC4Y 8JD (GB)**

(56) References cited:
**WO-A-92/14396          US-A- 3 587 588**
**US-A- 3 747 603          US-A1- 2005 070 949**
**US-A1- 2005 267 509**

EP 1 754 503 B1

**Description**

This invention relates to vaginal dilators, in particular vaginal dilators comprising spaced markings.

Vaginal dilators are used to treat a variety of conditions such as vaginismus, vaginal agenesis, or vaginal stenosis and are often used after vaginoplasty in order to assist shaping of the vagina.

Vaginismus is an involuntary contraction of the muscles surrounding the entrance to the vagina, making penetration impossible and/or painful. It is normal to treat this condition with vaginal dilators to allow the woman to overcome her fear of penetration in private.

Vaginal agenesis is a birth defect or congenital disorder which affects a relatively small proportion of women. It is primarily a physical abnormality in which the vagina stops developing. Typically sufferers have a short vagina (or neo-vagina) of maybe 3.5cm in length, but there may be no vagina at all.

Treatment of this condition may involve surgery but where surgery is required this is also usually followed by a period of vaginal training using appropriate dilators. In cases where non-surgical treatment is adequate, the "Frank" or the "Ingram" techniques are typical of the approaches adopted. These techniques utilise a medical dilator which is pushed into the vaginal cavity for sessions of about 20 to 30 minutes, perhaps several times daily. Using a series of dilators of increasing length and diameter, an acceptable vagina can often be created.

Vaginal stenosis is the narrowing and/or loss of flexibility of the vagina, often accompanied by other undesirable changes which are often a side effect of radiotherapy and/or genital surgery.

Radiation treatment may result in changes to the vagina such as the drying and thinning of the vaginal lining, fibrosis (formation of scar tissue), shortening and narrowing of the vagina, and reduction in the amount of vaginal lubrication. It is also likely to diminish the size and number of small blood vessels within the vagina. All of these side effects lead to vaginal stenosis and much drier, friable vaginal tissue.

As a means of reducing the effects of vaginal stenosis, patients are usually given vaginal dilators with which they exercise to increase blood circulation within the vaginal area. Estrogen creams are also often employed.

Vaginoplasty is a surgical procedure often used to create a neo-vagina. This may be required in order to treat vaginal agenesis but may also be employed for male to female gender changes.

vaginal dilation is a significant part of the postoperative recovery procedures, and although particularly intensive immediately after surgery, is likely to remain important for the remainder of the individual's life.

various dilators for other body lumens are known. In US 3587588, a cervical dilator is described to dilate the cervical canal at the top end of the vagina at the entry to the uterus. It is positioned by expert, not a patient, and requires vibration backwards and forwards at high frequencies. The maximum diameter of the cervical dilator is given in column 2, line 60 and is 4 to 11 mm.

In US 3747603 a cervical dilator having a handle and a probe is described. The probe has a maximum diameter of 12 mm and is used to reduce cervical damage during dilation procedures.

US2005/0070949 describes a transparent dilator which is used by a doctor in conjunction with an endoscope so that he can visualise the lumen being dilated. WO 00/41772 discloses a vaginal insert having rounded ends and a reduced cross sectional middle portion to excuse and improve the muscle tone of the feminine peluie floor.

Various vaginal dilator products are on the market. These usually take the form of a series of dilators of increasing diameters to be used as the treatment progresses. One such product sold comprises a series of hollow dilators of differing diameters which fit inside each other like Russian dolls. The dilators are used with a handle which clips into the end of each dilator.

These hollow dilators and the handle are, however, inherently unhygienic since it is very difficult to clean and subsequently dry the inside of each dilator or the handle. Moreover, since the dilators are hollow, they are very light and appear flimsy. They could perhaps be accidentally damaged and broken readily. It is also difficult to tell each diameter from the next diameter up or down in the series since all the dilators are the same colour, Moreover, since each dilator needs to fit inside the next dilator up in the series, all the dilators are of differing lengths and it is impossible for the user to determine how deeply penetration into the vagina has occurred. Furthermore, the top of handle is broad and likely to cause discomfort to the user.

Another product comprises a series of solid dilators of differing diameters but the same length some of which contain a series of identical regularly spaced rings. The rings are apparently for aesthetic appearance only as they are indistinguishable from one another by touch or sight and too small to feel by the user on vaginal entry. All the dilators are the same colour again making selection of the correct diameter of dilator problematic. Finally, it is again difficult for the user to determine how deeply penetration into the vagina has occurred. The user is required to hold the inserted dilator close to the vaginal opening, remove it carefully and measure the inserted length.

The inventor on this case has realised that with dilators currently on the market, it is impossible to determine how far into the vagina, the dilator has penetrated whilst dilation is in progress. For a patient trying to enlarge a vagina or neo-vagina, overcome vaginismus, or treat stenosis this can be critical. For example, in the treatment of agenesis, the aim is to create a vagina of sufficient size to accommodate an erect penis. If the user or therapist is unable to readily determine how deeply penetration has occurred she has no way of ensuring that the vagina she is creating is of sufficient length.

The present inventor has therefore devised a vaginal dilator comprising a plurality of spaced markings which the user or therapist can detect and distinguish by touch or sight when using the dilator. In this way, the user or therapist is able to determine how far the dilator has penetrated into the vagina without having to remove the dilator from the vagina.

Thus, viewed from one aspect the invention provides a vaginal dilator comprising a plurality of spaced markings wherein at least two types of marking are employed which are distinguishable from each other by touch and/or sight. Preferably, each marking is distinguishable from any markings adjacent thereto by touch and/or sight.

The vaginal dilator takes the form of a cylinder usually straight along its longest axis but conceivably slightly curved, with a rounded nose portion. Typically, each dilator has the same diameter throughout its length although sometimes the diameter of the dilator might vary along the length of the dilator for therapeutic reasons. Thus, the dilator might be wider towards the end away from the nose of the dilator.

The dilator can be made from any convenient material such as metal, glass or plastic, preferably plastic and is preferably solid (as opposed to hollow). Preferably, the material employed will have a medical approval for tissue contact use. Suitable materials are typically plastics and may be based on polyacetals, polyphenylenesulphones and polyetheretherketones. Certain materials sold under the Trade names Radel, Peek and Acetal/Delrin are of particular utility. The dilators can be machined or formed, e.g. by moulding, using well known methods.

The dilators of the invention preferably possess a nose portion which has been specifically designed to ease penetration into the vagina whilst also being blunt enough to reasonably minimize the risk of perforating the end of the vagina if undue force is exerted against the dilator whilst located within the vagina.

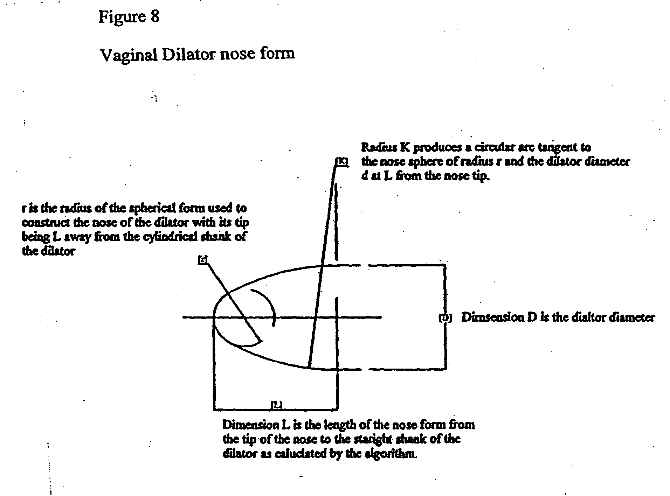

Whilst all dilators on the market possess a rounded nose portion, no other is believed possess a nose portion of identical shape to that of the preferred embodiment of the present invention. Thus, whilst a rounded nose portion, e.g. parabolic nose portion may be employed, it is preferred if the tip of the nose portion of the dilator of the invention is substantially hemispherical in shape, e.g. as shown in Figure 8. Thus, the tip of the dilator should be hemispherical, tapering of the nose portion beginning at the top and base of the hemisphere.

It is also preferred if the nose portion conforms to the following algorithm

$$L = f \times D$$

wherein D = Dilator diameter (mm)

L = Length (mm) of curved nose portion from tip to start of dilator diameter (i.e. the length of the nose portion)
f = Numeric factor in the range 1.0 to 1.3, preferably 1.05 to 1.2, especially 1.1 to 1.15, such as 1.1276.
(L is capped at 45mm to ensure the dilator does not possess an unacceptably long nose portion at higher diameters since such a long point would not dilate the end of the neo-vagina acceptably)

It is also preferred if the nose portion satisfies the equation

$$r = g \times L$$

r = Radius (mm) of ultimate spherical nose form (see figure 8)
g = Numeric factor 0.23 to 0.27, preferably 0.24 to 0.26, especially 0.2520
L = Length (mm) of curved nose portion from tip to start of dilator diameter.

(r is capped at 10, since the algorithm produces unacceptable dilators at larger (not often required) diameters).

The radius of the tangent needed to smoothly join the edge of the hemispherical tip with the flat edge of the overall dilator diameter is termed K (See Figure 8). In general the tangent traces a circular arc although a parabolic arc would also be acceptable. Typical values for K range from 60 to 80, e.g. 68 to 75 mm.

At least two different types of markings are used on the dilators of the invention, e.g. indented grooves and protruding rings, preferably at least three types of marking. By different types of marking is meant that the each marking type is distinguishable from another by touch and/or sight, preferably touch. Whilst it is within the scope of the invention to have identical markings adjacent to each other (e.g. two rings followed by two grooves then two rings etc) ideally, the spaced markings on the dilator are in a form which allows any adjacent markings to be distinguished from each other by touch and/or sight. Adjacent markings are those which are next to each other on the dilator (i.e. directly adjacent). It will be appreciated that the markings at the ends of the dilator are adjacent to only one other marking and should preferably be distinguishable from the marking adjacent thereto. By spaced markings is meant that the markings are distributed along at least a portion of the length of the dilator. By touch is meant that the hand/fingers/finger nail etc of the user can feel the markings and distinguish there between. If the dilator is being used by a therapist, the therapist may also be able to distinguish the markings through sight.

It is required that two different types of marking are used that are distinguishable by touch and/or sight. It is preferred if any adjacent markings are distinguishable, i.e. adjacent markings need to be different. It is within the scope of the invention however, for the same markings to be used alternately on the dilator. The minimum number of different markings required is therefore two although it will be appreciated that a higher number of different markings is preferred. For example, all markings on the dilator can be different.

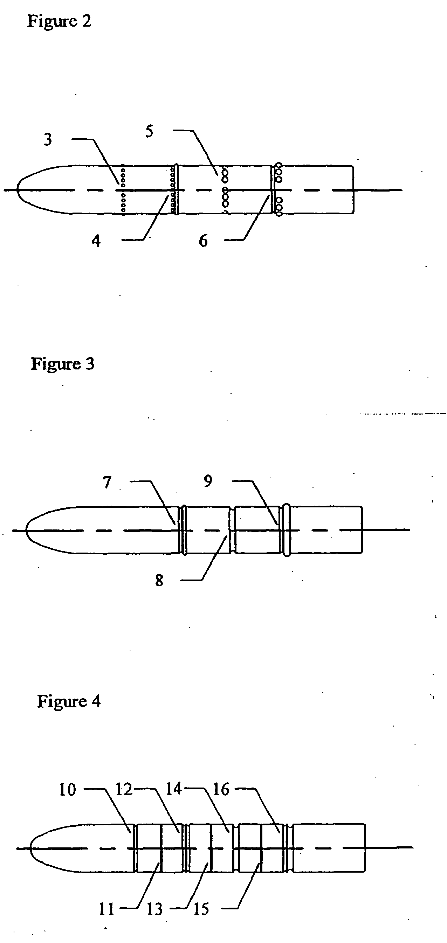

In a further embodiment, the 1st, 3rd, 5th etc markings are identical whilst 2nd, 4th, 6th etc markings are different (or vice versa). In this way, the fact that one set of markings is all the same is irrelevant since the user is able to determine which otherwise identical marking she is feeling/seeing by reference to the marking to which it is adjacent. Such an arrangement is depicted in Figure 4 as described further below.

The markings could take the form of indented rings or protruding ridges around the dilator. They might also take the form of a series of protrusions, e.g. hemispherical protrusions, or dimples on the dilator surface. varying shapes of protrusions might be used to distinguish between markings, e.g. prismatic, hemispherical, cuboid etc markings could be employed.

Markings could be grouped in twos or threes etc. Thus, one marking might take the form of two dimples then a gap then two dimples then a gap around the circumference of the dilator. The next marking could use three dimples and so on.

Rows of otherwise identical markings could also be repeated to enable the user to distinguish markings. Thus, a first marking might be a single ring, a second marking, 2 rings and so on. For the purposes of clarity, it will be clear that where for example two rings are used close together to distinguish from one ring or three rings etc, this constitutes a single marking.

The markings themselves might be of different sizes to enable the user to distinguish between them. Thinner and thicker ridges or indented rings could be used.

The person skilled in the art is able to devise all manner of ways of marking the dilator in accordance with the invention.

A further advantage of the markings of the invention is that they serve to retain lubricant which often has to be used to allow penetration of the dilator into the vaginal. Grooves in particular have been found to retain lubricant.

It is preferable if the markings on the dilator pass around its entire circumference although this is not essential. The markings might only partially go around the dilator circumference perhaps necessitating rotation of the dilator in order to find the feature.

The markings are preferably spaced in a pattern that the user is able to memorise. There might be a higher concentration of markings along certain portions of the dilator, e.g. at a typical vaginal penetration depth. Thus, there may be fewer markings at the two ends with more markings in the middle portion. The markings might therefore be spaced at the distances 5,7,9,10,11,12,14 cm along the dilator (from the nose).

Ideally, the markings are evenly spaced, e.g. at intervals of every 1 cm, every 1.25 cm, every 1.5cm, every 2 cm etc. Especially preferably, they are spaced apart by 12.5 mm.

It is preferred if the nose portion of the dilator is marking free. The first marking may be positioned at least 50 mm from the tip of the nose, e.g. at least 60 mm, ideally 70 to 100 mm from the nose, e.g. 85 to 90 mm.

The number of markings on the dilator may vary from 2 to 15, e.g. 5 to 12, preferably 8 to 10.

Markings can be introduced onto the dilator using any conventional method e.g. using moulds, lathes (for forming indented rings or ridges), etc.

As the patient proceeds through treatment, the diameter of, the dilator she should use will increase. It will be appreciated therefore that the invention also provides a kit comprising a series of dilators of differing diameters each possessing the dilator features described above. Whilst it is possible to use different patterns of markings on each dilator, it is preferred if each dilator within the kit has the same markings. This means that the user need remember only one pattern and also makes manufacture easier.

Since in such a series of dilators the differences in diameter from one dilator to the next in the series may be small and hence not readily determined by sight or feel, the invention also allows for the dilators to be colour coded. Thus, numbers 1,3,5,7 etc in a series may be one colour whilst numbers 2,4,6,8 etc may be another. It is believed that the difference in diameter from numbers 1 to 3 or 4 to 6 in a series would be clear by sight or feel alone. Alternatively, all dilators could possess different colours.

As their therapy progresses, patients with agensis will tend to require dilators of gradually increasing length since they tend to insert the entire length of the dilator into the neo-vagina. Thus, dilators can be of differing lengths (smaller diameter dilators will be shorter than larger diameter dilators and in the shorter dilators, markings around the circumference of the dilator are likely to be present nearer to the nose of the dilator).

It is preferred, however if all dilators are the same length. In a number of other products, it is observed that dilator length increases as dilator diameter increases. It is believed however that the use of dilators all of the same length is advantageous. A number of the smallest dilators in prior art products are so short that they are difficult to use. Also, by using dilators all of the same length, the same pattern of markings can be applied to the dilator.

The dilators may range from 120 to 250 mm in length, e.g. 140 to 220 mm in length, preferably 150 to 215 mm, especially about 162.5 mm. It will be appreciated however, that agensis patients may require dilators as small as 30 mm in length.

The diameters of the dilators may range from 15 mm to 50 mm, e.g. 20 mm to 45 mm, preferably 25 to 40 mm. Typically, each dilator may have a diameter 2 to 5 mm, e.g. 3 or 4 mm, greater than the previous dilator in the series. Thus, for dilators having diameters in the range 21 and 45 mm, with 3 mm increases, 9 dilators would be provided.

Ideally, the user should not touch the portion of the dilator which is inserted into the vaginal before or during use to help prevent any risk of infection. The dilators of the invention can therefore be provided with handles. The handle may be in any suitable form and may connect to the dilator by any conventional means. Thus, the handle and dilator could be screwed together using male and female threads or the handle and dilator could be clipped together, e.g. using a bayonet fitting. Attachment could be effected using magnets or using frictional engagement means. The person skilled in the art can develop numerous ways of attaching the dilator to the handle.

Since it should not be easy for the attachment between handle and dilator to come apart, a screw fitting is preferred.

The handle may take the form of an additional cylinder which can be attached to the dilator. It will be appreciated that it is preferred if the handle is adapted for use with all dilators in the series. The handle may also be spherical or disc shaped.

Especially preferably, the handle is adapted such that it can be gripped by the inner thigh of the user. It may be necessary for the dilator to remain in the vagina for a prolonged period. In this scenario, it is advantageous if the user can grip the dilator thus maintaining its presence within the vagina using the thighs thus leaving the hands free or enabling the user to adopt a potentially more comfortable position during the treatment.

A rounded disc shaped or spherical handle is especially useful in this embodiment.

Handles themselves can be adapted to connect to other handles, e.g. by providing a thread in the end of the handle.

It is also possible to manufacture the dilator with a handle integral therein. This makes the product slightly cheaper. The shape, size and nature of the handles will be as discussed above although this will come permanently attached the dilator.

The length of the handle portion may vary but is conveniently 30 to 70 mm in length, e.g. 40 to 60 mm, especially 50 mm. Some of the handles are likely to have a different diameter from the majority of the dilators in the kit of the invention.

The weight of the handle portion could also be varied to tailor the particular needs of the individual. A heavy handle may exert too great a moment on the vagina of certain users who might wish for a lighter handle.

A distancing portion could also be used to separate the handle from the end of the dilator. Thus, a cylindrical distancing portion adapted to connect to the dilator and adapted to connect to a handle may also be provided.

The vaginal dilators of the invention can be used in the treatment of any condition for which vaginal dilators are used. Examples of such conditions include vaginal agenesis, vaginal stenosis, vaginismus, vaginoplasty, post operative vaginal dilation etc.

Thus viewed from a further aspect the invention provides a method of treating a condition for which vaginal dilators are used, e.g. vaginal agenesis, vaginal stenosis, vaginismus, employing the dilators as hereinbefore described.

The invention will be further described with reference to the Figures.

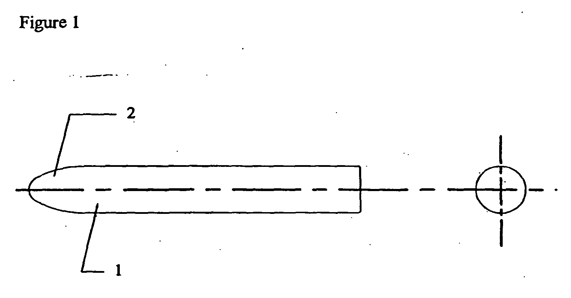

Figure 1 shows a typical vaginal dilator (1) of regular circular, cylindrical form. The nose of the dilator (2) is carefully designed to facilitate entry of the dilator into the vagina whilst also being blunt enough to reasonably minimize the risk of perforating the end of the vagina if undue force is exerted against the dilator whilst located within the vagina.

Figure 2 shows a cylindrical dilator with a number of tactile features added along the length of the dilator, of varying forms but at regular distances along the dilator from the rounded point of the dilator. The features comprise respectively:

(3) a single row of protruding small smoothly rounded raised bobbles completely encircling the dilator circumference;
(4) a single row of recessed smoothly curved dimples closely followed by a small protruding raised ring, both extending round the full circumference of the dilator;
(5) a selection of slightly larger circular dimples, in this case in threes, around the complete circumference of the dilator;
(6) a recessed smoothly rounded ring followed by a selection of larger protruding smoothly rounded bobbles, in threes, around the circumference of the dilator.

Figure 3 shows a cylindrical dilator with a number of tactile features added along the length of the dilator, of varying forms but at regular distances along the dilator from the rounded point of the dilator. The features comprise respectively:

(7) A smoothly rounded recessed ring passing around the complete circumference of the dilator closely followed by a similar sized protruding circular ring passing round the complete circumference of the dilator;
(8) A single, somewhat larger recessed smoothly rounded ring passing around the complete circumference of the dilator;
(9) A single recessed smoothly rounded ring passing round the complete circumference of the dilator followed by a somewhat larger, protruding smoothly rounded ring passing round the complete circumference of the dilator.

Figure 4 shows a cylindrical dilator with a number of tactile features added along the length of the dilator, of varying forms but at regular distances along the dilator from the rounded point of the dilator. The features comprise respectively:

(10) a single small recessed smoothly rounded ring passing around the complete circumference of the dilator;
(11) a single narrow recessed ring of angular form passing around the complete circumference of the dilator;
(12) A set to two small recessed smoothly rounded rings passing around the complete circumference of the dilator;
(13) A single narrow recessed ring of angular form passing around the complete circumference of the dilator;
(14) A single larger recessed smoothly rounded ring passing around the complete circumference of the dilator;
(15) A single narrow recessed ring of angular form passing around the complete circumference of the dilator;
(16) A set to two smoothly rounded recessed rings, one of a small dimension followed by another larger one with both passing around the complete circumference of the dilator.

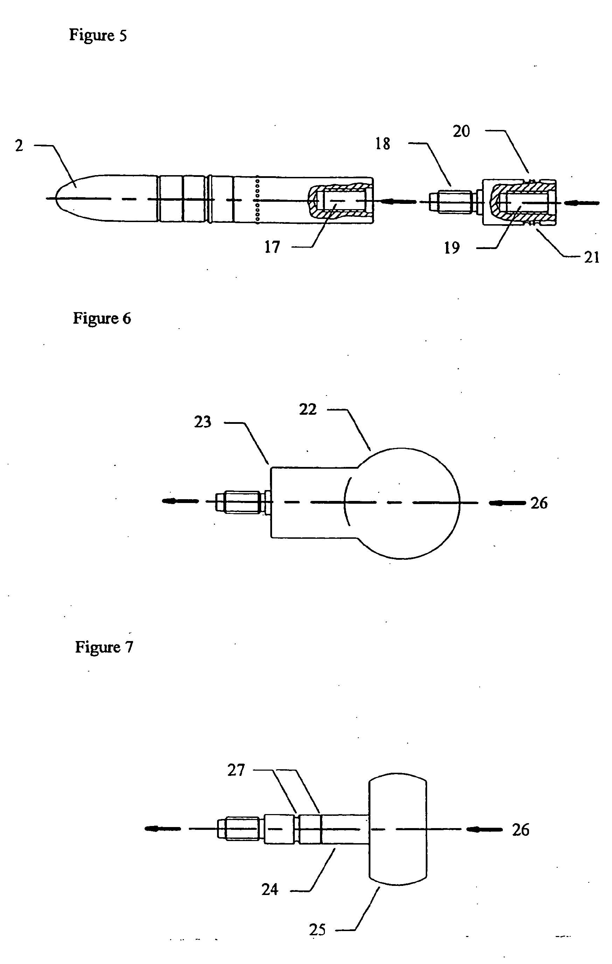

Figure 5 shows a cylindrical dilator with a number of different tactile features added along the dilator (not more fully described here) at various distances from the nose of the dilator. A recessed screw thread (17) is included within the end of the dilator (1) furthest from the nose portion (2). This screw thread and appropriate locating lugs and recesses allow the apparatus to be assembled with a variety of different handles/distancing portions. An example of a male thread and appropriate locating lugs and recesses (18) is shown. A further recessed thread (19) at the end of the handle/distancing portion allows it to be assembled with other handles if desired.

The handle/distancing portion (20) may be provided with grooves (21) to enable the user to grip the handle better.

Figure 6 shows an example of a spherical handle (22) in which a larger wider portion is distanced by a cylinder (23) from the end of the dilator, or other handle, into which it is assembled. The spherical portion of this handle makes it easy and comfortable for the user to grip it with their inner thighs when assembled with a dilator which has been inserted into their vagina. This handle may also have another recessed thread at its end (26) which allows it to be assembled with other handles if desired. Such a thread might be recessed on a small flat surface added onto the end of the sphere furthest from the thread shown.

Figure 7 shows an example of a cylindrical handle distance piece elliptical disc (24) on which a larger smoothly rounded larger circular or perhaps elliptical disc portion (25) is included. This handle may make it easier for the user to grip it with their inner thighs when assembled with a dilator which has been inserted into their vagina.

The distancing part of the handle (24) has a narrow recessed smoothly rounded groove followed by an angular recessed ring (27), both running around the circumference of the distancing portion of the handle in order to facilitate either the user gripping it whether or not it is assembled with another handle or a dilator, and whether or not it has been inserted into a vagina.

This handle may also have another recessed thread at its end (26) which allows it to be assembled with other handles if desired. Such a thread might be recessed on the small flat surface added onto the end of the disc furthest from the thread shown.

Figure 8 shows the nose portion (2) of a dilator and depicts the hemisphere present at its tip (28). Algorithm variables L, K, r and D are shown.

**Claims**

1. A vaginal dilator (1) in the form of a cylinder with a rounded nose portion comprising a plurality of spaced markings (3;4;5;6;7;8;9;10;11;12;13;14;15; 16) **characterised by** at least two types of marking being employed which are distinguishable from each other by touch and/or sight.

2. A dilator as claimed in claim 1 wherein each marking is distinguishable from any markings adjacent thereto by touch and/or sight.

3. A dilator as claimed in claim 1 or 2 having a nose portion (2), the tip of said nose portion being substantially hemispherical in shape.

4. A dilator as claimed in claim 3 wherein the nose portion conforms to the following algorithm

$$L = f \times D$$

wherein D = Dilator diameter (mm)
L = Length (mm) of curved nose portion from tip to start of dilator diameter (i.e. the length of the nose portion)
f = Numeric factor in the range 1.0 to 1.3

5. A dilator as claimed in any preceding claim wherein the nose portion satisfies the equation

$$r = g \times L$$

r = Radius (mm) of ultimate spherical nose form
g = Numeric factor 0.23 to 0.27
L = Length (mm) of curved nose portion from tip to start of dilator diameter.

6. A dilator as claimed in any preceding claim wherein said markings comprise indented grooves (6;7;8; 910;;11;12;13;14;15;16).

7. A dilator as claimed in any preceding claim wherein markings pass all the way around the circumference of the dilator.

8. A dilator as claimed in any preceding claim wherein markings are evenly spaced.

9. A dilator as claimed in any preceding claim wherein the nose portion is marking free.

10. A dilator as claimed in any preceding claim wherein the dilator comprises 2 to 15 markings.

11. A dilator as claimed in any preceding claim further comprising a handle.

12. A dilator as claimed in claim 11 wherein said handle is spherical or disc shaped.

13. A dilator as claimed in any preceding claim having a diameter of 15 to 50 mm.

14. A dilator as claimed in any preceding claim wherein at least two types of marking are employed which are distinguishable from each other by touch.

15. A dilator as claimed in any preceding claim which is solid.

16. A kit comprising a plurality of vaginal dilators as claimed in claim 1 to 15.

17. A kit as claimed in claim 16 wherein the 1st (smallest), 3rd, 5th etc dilators are one colour and the 2nd, 4th, 6th etc dilators are another colour.

**Patentansprüche**

1. Scheidendilatator (1) in Form eines Zylinders mit einem abgerundeten Nasenabschnitt, der mehrere in Abständen angeordnete Markierungen (3;4;5;6;7;8; 9;10;11;12;13;14;15;16) umfasst, **dadurch gekennzeichnet, dass** mindestens zwei Arten von Markierungen verwendet werden, die durch Tastgefühl und/oder Sicht voneinander unterscheidbar sind.

2. Dilatator nach Anspruch 1, wobei die Markierungen jeweils durch Tastgefühl und/oder Sicht von etwaigen dazu benachbarten Markierungen unterscheidbar sind.

3. Dilatator nach Anspruch 1 oder 2 mit einem Nasenabschnitt (2), wobei die Spitze des genannten Nasenabschnitts im Wesentlichein halbkugelförmig ist.

4. Dilatator nach Anspruch 3, wobei der Nasenabschnitt dem folgenden Algorithmus entspricht:

$$L = f \times D$$

wobei D = Dilatatordurchmesser (mm)
L = Länge (mm) des gekrümmten Nasenabschnitts von der Spitze bis zum Anfang des Dilatatordurchmesser (d.h. die Länge des Nasenabschnitts)
f = numerischer Faktor im Bereich 1,0 bis 1,3.

5. Dilatator nach einem der vorangehenden Ansprüche, wobei der Nasenabschnitt folgende Gleichung erfüllt:

$$r = g \times L$$

r = Radius (mm) der endgültigen kugelförmigen Nasenform
g = numerischer Faktor 0,23 bis 0,27
L = Länge (mm) des gekrümmten Nasenabschnitts von der Spitze bis zum Anfang des Dilatatordurchmessers.

6. Dilatator nach einem der vorangehenden Ansprüche, wobei die genannten Markierungen eingeschnittene Rillen (6;7;8;9;10;11;12;13;14;15;16) umfassen.

7. Dilatator nach einem der vorangehenden Ansprüche, wobei Markierungen vollständig um den Umfang des Dilatators herum verlaufen.

8. Dilatator nach einem der vorangehenden Ansprüche, wobei die Markierungen in gleichmäßigen Abständen angeordnet sind.

9. Dilatator nach einem der vorangehenden Ansprüche, wobei der Nasenabschnitt frei von Markierungen ist.

**10.** Dilatator nach einem der vorangehenden Ansprüche, wobei der Dilatator 2 bis 15 Markierungen umfasst.

**11.** Dilatator nach einem der vorangehenden Ansprüche, der weiter einen Griff umfasst.

**12.** Dilatator nach Anspruch 11, wobei der genannten Griff kugelförmig oder kreisscheibenförmig ist.

**13.** Dilatator nach einem der vorangehenden Ansprüche mit einem Durchmesser von 15 bis 50 mm.

**14.** Dilatator nach einem der vorangehenden Ansprüche, wobei mindestens zwei Arten von Markierungen verwendet werden, die durch Tastgefühl voneinander unterscheidbar sind.

**15.** Dilatator nach einem der vorangehenden Ansprüche, der massiv ist.

**16.** Set, das mehrere Scheidendilatatoren nach Ansprüchen 1 bis 15 umfasst.

**17.** Set nach Anspruch 16, wobei der 1. (kleinste), 3., 5. usw. Dilatator eine Farbe aufweisen und der 2., 4., 6. usw. Dilatator eine andere Farbe aufweisen.


**Revendications**

**1.** Dilatateur vaginal (1) sous forme de cylindre présentant une partie de nez arrondi comprenant une pluralité de repères espacés (3 ; 4 ; 5 ; 6 ; 7 ; 8 ; 9 ; 10 ; 11 ; 12 ; 13 ; 14 ; 15 ; 16) **caractérisé par le fait qu'**au moins deux types de repères sont employés et sont différenciables l'un de l'autre au toucher et/ou à la vue.

**2.** Dilatateur selon la revendication 1, dans lequel chaque repère est différenciable des repères qui lui sont adjacents au toucher et/ou à la vue.

**3.** Dilatateur selon la revendication 1 ou 2, ayant une partie de nez (2), la pointe de ladite partie de nez étant sensiblement de forme hémisphérique.

**4.** Dilatateur selon la revendication 3, dans lequel la partie de nez répond à l'algorithme suivant

$$L = f \times D$$

où D = diamètre du dilatateur (mm)
L = longueur (mm) de la partie de nez courbe depuis la pointe jusqu'au début du diamètre du dilatateur (c.-à-d. la longueur de la partie de nez)

f = facteur numérique dans la plage 1,0 à 1,3.

**5.** Dilatateur selon l'une quelconque des revendications précédentes, dans lequel la partie de nez satisfait l'équation

$$r = g \times L$$

r = rayon (mm) d'une forme de nez sphérique parfaite
g = facteur numérique de 0,23 à 0,27
L = longueur (mm) de la partie de nez courbe depuis la pointe jusqu'au début du diamètre du dilatateur.

**6.** Dilatateur selon l'une quelconque des revendications précédentes, dans lequel lesdits repères comprennent des rainures creuses (6 ; 7 ; 8 ; 9 ; 10 ; 11 ; 12 ; 13 ; 14 ; 15 ; 16).

**7.** Dilatateur selon l'une quelconque des revendications précédentes, dans lequel les repères parcourent tout le tour de la circonférence du dilatateur.

**8.** Dilatateur selon l'une quelconque des revendications précédentes, dans lequel les repères sont équidistants.

**9.** Dilatateur selon l'une quelconque des revendications précédentes, dans lequel la partie de nez est dépourvue de repère.

**10.** Dilatateur selon l'une quelconque des revendications précédentes, dans lequel le dilatateur comprend de 2 à 15 repères.

**11.** Dilatateur selon l'une quelconque des revendications précédentes, comprenant en outre une poignée.

**12.** Dilatateur selon la revendication 11, dans lequel ladite poignée est de forme sphérique ou discale.

**13.** Dilatateur selon l'une quelconque des revendications précédentes, ayant un diamètre de 15 à 50 mm.

**14.** Dilatateur selon l'une quelconque des revendications précédentes, dans lequel au moins deux types de repères sont employés, lesquels sont différenciables l'un de l'autre au toucher.

**15.** Dilatateur selon l'une quelconque des revendications précédentes, lequel est solide.

**16.** Kit comprenant une pluralité de dilatateurs vaginaux selon les revendications 1 à 15.

**17.** Kit selon la revendication 16, dans lequel les premier (le plus petit), troisième, cinquième, etc., dilatateurs sont d'une couleur et les deuxième, quatrième, sixième etc. dilatateurs sont d'une autre couleur.

Figure 1

Figure 2

Figure 3

Figure 4

Figure 5

Figure 6

Figure 7

Figure 8

Vaginal Dilator nose form

Radius K produces a circular arc tangent to
[K] the nose sphere of radius r and the dilator diameter
d at L from the nose tip.

r is the radius of the spherical form used to
construct the nose of the dilator with its tip
being L away from the cylindrical shank of
the dilator

[r]

[D] Dimension D is the dialtor diameter

[L]

Dimension L is the length of the nose form from
the tip of the nose to the staright shank of the
dilator as caluclated by the algorithm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3587588 A
- US 3747603 A
- US 20050070949 A
- WO 0041772 A